# EUROPEAN PATENT APPLICATION

(11) **EP 4 404 204 A2**
(43) Date of publication of application: **24.07.2024**
(21) Application number: 23186668.2
(22) Date of filing: 20.07.2023
(51) Int. Cl.: G16C 10/00

(54) **APPARATUS AND METHOD WITH DATA PROCESSING**

(30) Priority: 17.01.2023 KR 20230006767
(71) Applicant: Samsung Electronics Co., Ltd., Suwon-si, Gyeonggi-do 16677 (KR)
(72) Inventor: Bae, Geuntae, Suwon-si, Gyeonggi-do (KR); Kim, Kiha, Suwon-si, Gyeonggi-do (KR); Park, Keunmo, Suwon-si, Gyeonggi-do (KR)
(74) Representative: Grünecker Patent- und Rechtsanwälte PartG mbB

(57) **Abstract**

A data processing apparatus and data processing method are provided. The data processing apparatus includes one or more processors configured to execute instructions, a memory configured to store the instructions that, when executed by the one or more processors, configure the one or more processors to determine first operation result data using a computational algorithm in a first predetermined time period and predict second predicted result data of a set second time period using a machine learning model provided the first operation result data; and determine third operation result data using the computational algorithm in a set third time period after the second time period.

## Description

### BACKGROUND

### 1. Field

The following description relates to an apparatus and method with data processing.

### 2. Description of Related Art

Molecular dynamics simulation simulates a temporal change process in the entire physical system by calculating a force acting on each particle over time and continuously calculating a position and movement velocity of the particles. Molecular dynamics simulation may utilize a large amount of data processing operations because an operation process for each of numerous particles and an operation process for the interaction between the particles is desired. Typically, a plurality of processors may be synchronized to perform data processing in parallel.

### SUMMARY

This Summary is provided to introduce a selection of concepts in a simplified form that are further described below in the Detailed Description. This Summary is not intended to identify key features or essential features of the claimed subject matter, nor is it intended to be used as an aid in determining the scope of the claimed subject matter.

The invention is claimed in the independent claims. Preferred embodiments are specified in the dependent claims.

In a general aspect, a data processing apparatus includes one or more processors configured to execute instructions; a memory configured to store the instructions that, when executed by the one or more processors, configure the one or more processors to determine first operation result data using a computational algorithm in a first predetermined time period; predict second predicted result data of a set second time period using a machine learning model provided the first operation result data; and determine third operation result data using the computational algorithm in a set third time period after the second time period.

The second time period may include a plurality of time periods, wherein the second predicted result data comprises predicted result data corresponding to each of the plurality of time periods, and wherein an overall data processing speed may increase in proportion to a number of time periods comprised in the second time period.

The first operation result data may correspond to time series data.

The machine learning model may generate the predicted result data based on a polynomial approximation algorithm.

The one or more processors may be configured to calculate motions of particles in a molecular dynamics simulation operation based on the computational algorithm.

The one or more processors may be configured to train the machine learning model based on the first operation result data; and determine whether a first condition is satisfied based on the first operation result data, and the determining of the second predicted result data is performed in response to the first condition being determined to be satisfied.

The one or more processors may be configured to determine second operation result data based on the first operation result data and the computational algorithm in the second time period in response to the first condition being determined to not be satisfied.

The one or more processors may be configured to determine a first loss value of the machine learning model based on the first operation result data, and the first condition is satisfied in response to the first loss value being less than a threshold value.

The one or more processors may be configured to determine a second loss value of the machine learning model based on the second predicted result data; determine whether a second condition is satisfied based on the second predicted result data; determine third operation result data based on the second predicted result data and the computational algorithm in a third time period in response to the second condition being determined to not be satisfied; and determine third predicted result data corresponding to the third operation result data based on the second predicted result data and the machine learning model in the third time period in response to the second condition being determined to be satisfied, and the second condition is satisfied in response to the second loss value being less than the threshold value.

In a general aspect, a data processing apparatus includes one or more processors configured to execute instructions; a memory, configured to store the instructions that when executed by the one or more processors, configure the one or more processors to determine first operation result data based on a computational algorithm in a first predetermined time period; predict second predicted input data of a set second time period and third predicted input data of a set third time period using a machine learning model provided the first operation result data; predict second predicted result data based on the second predicted input data and the computational algorithm in the second time period; and predict third predicted result data based on the third predicted input data and the computational algorithm in the third time period, wherein the determining of the second predicted result data in the second time period, and the determining of the third predicted result data in the third time period are performed simultaneously and in parallel.

The second time period may include a plurality of time periods, the second predicted result data may include predicted result data corresponding to each of the plurality of time periods, the third time period may include a plurality of time periods, the third predicted result data may include predicted result data corresponding to each of the plurality of time periods of the third time period, and an overall data processing speed increases in proportion to the number of time periods included in the second time period and the number of time periods included in the third time period.

In a general aspect, a processor-implemented data processing method performed by a data processing apparatus, includes determining first operation result data based on a computational algorithm in a first time period; and predicting second predicted result data of a second time period using a machine learning model provided the first operation result data.

The second time period may include a plurality of time periods, wherein the second predicted result data comprises predicted result data corresponding to each of the plurality of time periods, and wherein an overall data processing speed increases in proportion to a number of time periods comprised in the second time period.

The first operation result data may correspond to time series data.

The machine learning model may generate the predicted result data based on a polynomial approximation algorithm.

The method may further include calculating motions of particles in a molecular dynamics simulation operation based on the computational algorithm.

The method may further include training the machine learning model based on the first operation result data, wherein the prediction of the second predicted result data may include determining whether a first condition is satisfied based on the first operation result data; and selecting to perform the predicting of the second predicted result data in response to the first condition being determined to be satisfied.

The method may further include determining second operation result data based on the first operation result data and the computational algorithm in the second time period in response to the first condition being determined to not be satisfied.

The method may further include determining a first loss value of the machine learning model based on the first operation result data, wherein the first condition is satisfied in response to the first loss value being less than a threshold value.

The method may further include determining whether a second condition is satisfied based on the second predicted result data; determining third operation result data based on the second predicted result data and the computational algorithm in a third time period in response to the second condition being determined to not be satisfied; and determining third predicted result data corresponding to the third operation result data based on the second predicted result data and the machine learning model in the third time period in response to the second condition being determined to be satisfied.

The machine learning model may be a multi-layer perceptron.

In a general aspect, a data processing apparatus includes one or more processors configured to execute instructions; a memory configured to store the instructions that, when executed by the one or more processors, configure the one or more processors to: determine first operation result data using a computational algorithm in a first predetermined time period; predict second predicted result data of a set second time period using a machine learning model provided the first operation result data; and determine second operation result data based on the first operation data using the computational algorithm in the second time period.

Other features and aspects will be apparent from the following detailed description, the drawings, and the claims.

### BRIEF DESCRIPTION OF DRAWINGS

FIG. 1 illustrates an example overview of an example data processing apparatus, in accordance with one or more embodiments.
FIG. 2 illustrates an example configuration of an example electronic device, in accordance with one or more embodiments.
FIG. 3 illustrates an example operation of an example machine learning model, in accordance with one or more embodiments.
FIG. 4 illustrates an example molecular dynamics simulation, in accordance with one or more embodiments.
FIG. 5 illustrates an example molecular dynamics simulation, in accordance with one or more embodiments.
FIG. 6 illustrates an example data processing method, in accordance with one or more embodiments.
FIG. 7 illustrates an example operation of an example data processing apparatus conditionally determining predicted result data, in accordance with one or more embodiments.
FIG. 8 illustrates an example data processing method in which an example data processing apparatus performs a sequential mode and an acceleration mode, in accordance with one or more embodiments.
FIG. 9 illustrates an example operation of an example data processing apparatus determining multiple predicted result data in parallel, in accordance with one or more embodiments.

Throughout the drawings and the detailed description, unless otherwise described or provided, the same drawing reference numerals may be understood to refer to the same or like elements, features, and structures. The drawings may not be to scale, and the relative size, proportions, and depiction of elements in the drawings may be exaggerated for clarity, illustration, and convenience.

### DETAILED DESCRIPTION

The following detailed description is provided to assist the reader in gaining a comprehensive understanding of the methods, apparatuses, and/or systems described herein. However, various changes, modifications, and equivalents of the methods, apparatuses, and/or systems described herein will be apparent after an understanding of the disclosure of this application. For example, the sequences within and/or of operations described herein are merely examples, and are not limited to those set forth herein, but may be changed as will be apparent after an understanding of the disclosure of this application, except for sequences within and/or of operations necessarily occurring in a certain order. As another example, the sequences of and/or within operations may be performed in parallel, except for at least a portion of sequences of and/or within operations necessarily occurring in an order, e.g., a certain order. Also, descriptions of features that are known after an understanding of the disclosure of this application may be omitted for increased clarity and conciseness.

The features described herein may be embodied in different forms and are not to be construed as being limited to the examples described herein. Rather, the examples described herein have been provided merely to illustrate some of the many possible ways of implementing the methods, apparatuses, and/or systems described herein that will be apparent after an understanding of the disclosure of this application. The use of the term "may" herein with respect to an example or embodiment, e.g., as to what an example or embodiment may include or implement, means that at least one example or embodiment exists where such a feature is included or implemented, while all examples are not limited thereto. The use of the terms "example" or "embodiment" herein have a same meaning, e.g., the phrasing "in one example" has a same meaning as "in one embodiment", and "one or more examples" has a same meaning as "in one or more embodiments."

The terminology used herein is for describing various examples only and is not to be used to limit the disclosure. The articles "a," "an," and "the" are intended to include the plural forms as well, unless the context clearly indicates otherwise. As used herein, the term "and/or" includes any one and any combination of any two or more of the associated listed items. As nonlimiting examples, terms "comprise" or "comprises," "include" or "includes," and "have" or "has" specify the presence of stated features, numbers, operations, members, elements, and/or combinations thereof, but do not preclude the presence or addition of one or more other features, numbers, operations, members, elements, and/or combinations thereof, or the alternate presence of an alternative stated features, numbers, operations, members, elements, and/or combinations thereof. Additionally, while one embodiment may set forth such terms "comprise" or "comprises," "include" or "includes," and "have" or "has" specify the presence of stated features, numbers, operations, members, elements, and/or combinations thereof, other embodiments may exist where one or more of the stated features, numbers, operations, members, elements, and/or combinations thereof are not present.

Throughout the specification, when a component or element is described as being "on", "connected to," "coupled to," or "joined to" another component, element, or layer it may be directly (e.g., in contact with the other component, element, or layer) "on", "connected to," "coupled to," or "joined to" the other component, element, or layer or there may reasonably be one or more other components, elements, layers intervening therebetween. When a component, element, or layer is described as being "directly on", "directly connected to," "directly coupled to," or "directly joined" to another component, element, or layer there can be no other components, elements, or layers intervening therebetween. Likewise, expressions, for example, "between" and "immediately between" and "adjacent to" and "immediately adjacent to" may also be construed as described in the foregoing.

Although terms such as "first," "second," and "third", or A, B, (a), (b), and the like may be used herein to describe various members, components, regions, layers, or sections, these members, components, regions, layers, or sections are not to be limited by these terms. Each of these terminologies is not used to define an essence, order, or sequence of corresponding members, components, regions, layers, or sections, for example, but used merely to distinguish the corresponding members, components, regions, layers, or sections from other members, components, regions, layers, or sections. Thus, a first member, component, region, layer, or section referred to in the examples described herein may also be referred to as a second member, component, region, layer, or section without departing from the teachings of the examples.

As used herein, the term "and/or" includes any one and any combination of any two or more of the associated listed items. The phrases "at least one of A, B, and C", "at least one of A, B, or C", and the like are intended to have disjunctive meanings, and these phrases "at least one of A, B, and C", "at least one of A, B, or C", and the like also include examples where there may be one or more of each of A, B, and/or C (e.g., any combination of one or more of each of A, B, and C), unless the corresponding description and embodiment necessitates such listings (e.g., "at least one of A, B, and C") to be interpreted to have a conjunctive meaning.

Unless otherwise defined, all terms, including technical and scientific terms, used herein have the same meaning as commonly understood by one of ordinary skill in the art to which this disclosure pertains and specifically in the context on an understanding of the disclosure of the present application. Terms, such as those defined in commonly used dictionaries, are to be interpreted as having a meaning that is consistent with their meaning in the context of the relevant art and specifically in the context of the disclosure of the present application, and are not to be interpreted in an idealized or overly formal sense unless expressly so defined herein.

Example embodiments may include large-scale distributed learning in the field of deep learning. In an example, in scientific computational simulation in various application fields, such as, but not limited to, computational physics, computational chemistry, and biology, applications in which dozens to thousands of server clusters perform a single job are implemented. In particular, molecular dynamics simulation, which simulates a process in which multiple particles interact with each other and change over time in a two-dimensional (2D) or three-dimensional (3D) physical system using Newtonian dynamics, is a representative application field of high-performance computing (HPC) using parallel data processing.

When processors perform data processing in parallel for a single job, synchronization of the processes with each other, and the exchange of intermediate data may be desired.

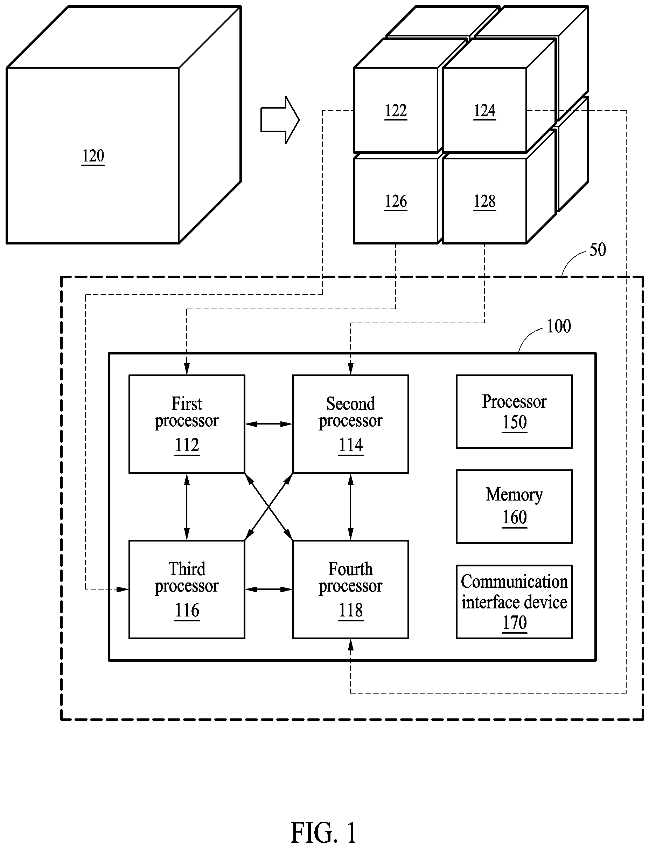

FIG. 1 illustrates an example overview of an example data processing apparatus, in accordance with one or more embodiments.

Referring to FIG. 1, an example electronic device 50 may include one or more example data processing apparatuses (or hardware system(s)) 100, and may be apparatuses that perform data processing, and may perform, as a non-limited example, high performance computing (HPC) that performs parallel operations using a large number of computational resources. For example, the data processing apparatus(es) 100 may perform or control multi-node distributed computing in which several server resources are implemented in the form of one or more clusters on a network in order to quickly perform a molecular dynamics simulation that uses a large number of operation processes. Additionally, the data processing apparatus 100 may perform or control applications that utilize large-scale operations, such as, as only examples, deep learning, weather prediction simulation, scientific computational simulation, and physical property analysis. The one or more data processing apparatus(es) 100 may be a distributed processing system of distributed servers, for example.

The data processing apparatus 100 may include a plurality of processors 112, 114, 116, and 118 that perform various operations. In an example, each processor 112, 114, 116, and 118 may be a distributed server. The example of FIG. 1 illustrates four processors 112, 114, 116, and 118 for ease of description. However, the examples are not limited thereto. In examples, the data processing apparatus 100 may include two or more processors that perform parallel operations, and the number of processors are not limited. In an example, the data processing apparatus 100 may further include a central processor 150 that controls the various respective processors 112, 114, 116, and 118. The data processing apparatus 100 may further include a memory 160 and communication interface device 170.

Each of the processors 112, 114, 116, and 118 may perform a parallel operation with another processor for a given job, and synchronize operations with other processors to perform parallel processing. In an example, the second processor 114 may synchronize operations with the first processor 112 to perform parallel processing for a given job. Each of the processors 112, 114, 116, and 118 may include hardware components of a processor (e.g., a central processing unit (CPU), a graphics processing unit (GPU), a neural processing unit (NPU), and/or a field programmable gate array (FGPA)).

In an example, when the data processing apparatus 100 performs molecular dynamics simulation, the data processing apparatus 100 may simulate a process in which multiple particles interact with each other and change over time in a 3D system 120 using Newtonian dynamics. The data processing apparatus 100 may perform parallel processing by implementing the plurality of processors 112, 114, 116, and 118 in order to perform a large number of operations more quickly in a parallel manner. The processors 112, 114, 116, and 118 may perform parallel processing in which jobs are divided and simultaneously processed, and a space division scheme may be used for the parallel processing. The space division scheme may be a scheme in which the 3D system 120 (e.g., a virtual system) corresponding to the entire simulation space is divided by the number of computational resources (e.g., processors) and each divided space (e.g., sub-systems 122, 124, 126, and 128) is simultaneously processed by each computational resource.

Each of the processors 112, 114, 116, and 118 may calculate motions of particles for molecular dynamics simulation using a computational algorithm, and distribute and process the calculation for a predetermined sub-system in the 3D system 120. For example, the first processor 112 may control operations in the first sub-system 126, the second processor 114 may control operations in the second sub-system 128, the third processor 116 may control operations in the third sub-system 122, and the fourth processor 118 may control operations in the fourth sub-system 124. In an example, processors other than the processors 112, 114, 116, and 118 may control operations in sub-systems other than the sub-systems 122, 124, 126, and 128. The processors 112, 114, 116, and 118 may independently perform operations on the motions of particles in the corresponding sub-systems 126, 128, 122, and 124 according to the computational algorithm, and perform communication in a mutually synchronized manner when information exchange is desired in a boundary area between the subsystems 122, 124, 126, and 128.

During data processing, the plurality of processors may exchange intermediate operation results for communication. Communication between the first processor 112 and the second processor 114 may have to be performed in synchronization with each other, and the first processor 112 and the second processor 114 may have to be interlocked to share operation results for other particles (e.g., with both the first processor 112 and the second processor 114 being connected to a shared interlock hardware). Parallel processing between processors may be efficiently performed by reducing the number of data transmission and reception operations by performing data prediction using patterns of communication data or by reducing the amount of data transmission by compressing and transmitting data.

Each of the processors 112, 114, 116, and 118 may sequentially process time series data. For example, a processor may sequentially process information (e.g., position, acting force, velocity) necessary for calculating the motion of particles included in the sub-system through a plurality of time periods. After completing data processing in an arbitrary time period, the processor may process data in a next time period based on a data result generated as a result of the data processing in the arbitrary time period. Through such sequential processing, the processor may process data in a subsequent time period only when data processing in a previous time period is completed, and thus, a delay may occur in the entire data processing process.

The data processing apparatus and data processing method according to an example may improve overall data processing speed and data processing efficiency by omitting or predicting a part of a process of sequentially processing time series data.

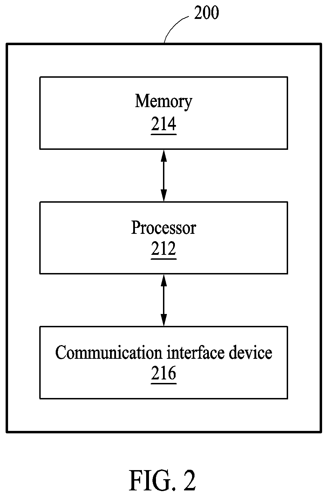

FIG. 2 illustrates an example configuration of an example computing device, in accordance with one or more embodiments.

Referring to FIG. 2, a computing device 200 may include a processor 212, a memory 214, and a communication interface device 216. In an example, the computing device 200 may correspond to one of the processors 112, 114, 116, and 118 of FIG. 1. In an example, each of the processors 112, 114, 116, and 118 is a computing device 200. In an example, the computing device200 may correspond to the data processing apparatus 100 of FIG. 1 and the processor 212 may be representative of processors 112, 114, 116, and 118. In an example, the processor 212, memory 214, and the communication device may respectively correspond to processor 150, memory 160, and communication interface device 170 of FIG. 1.

The processor 212 may control other components (e.g., hardware or software components) of the computing device200 and may perform various types of data processing or operations. As at least a part of data processing or operations, the processor 212 may store instructions or data received from another component in the memory 214, process the instructions or data stored in the memory 214, and store result data in the memory 214. Operations performed by the processor 212 may be substantially the same as operations performed by the processor 200 or the data processing apparatus 100.

The memory 214 may store information necessary for the processor 212 to perform a processing operation. For example, the memory 214 may store instructions to be executed by the processor 212 and may store related information while a software or a program is executed in the processor 200. The memory 214 may include a volatile memory such as a random-access memory (RAM), a dynamic RAM (DRAM), and a static RAM (SRAM), and/or a non-volatile memory known in the art such as a flash memory.

The communication interface device 216 may communicate with other processors. The communication interface device 216 may exchange data with other processors through a communication link (e.g., NVLINK).

The memory 214 may include instructions to execute a machine learning model. The machine learning model may be executed under the control of the processor 212. The processor 212 may transmit training data to train the machine learning model and a control signal to learn the machine learning model to the memory 214. The machine learning model may be trained based on the training data transmitted from the processor 212 and may output predicted result data for input data under the control of the processor 212.

In an example, the machine learning model may be implemented as a multi-layer perceptron, but the type of the machine learning model is not limited thereto. The machine learning model may determine predicted result data based on a polynomial approximation algorithm, but the algorithm to determine the predicted result data by the machine learning model may vary according to examples.

The processor 212 may determine first operation result data by implementing a computational algorithm. The computational algorithm may be, as an example, a Verlet integration algorithm. However, the type of the computational algorithm is not limited thereto and may vary according to examples. The processor 212 may calculate motions of particles for molecular dynamics simulation using such a computational algorithm. The first operation result data may correspond to motion information (e.g., position, acting force, velocity) necessary for calculating the motion of corresponding particles in a first time period in the molecular dynamics simulation. The first operation result data may correspond to time series data.

The processor 212 may transmit the first operation result data to the memory 214. The processor 212 may determine second predicted result data of a second time period based on the first operation result data and the machine learning model. The second time period may be a time period after the first time period. In an example, the second time period may include a plurality of time periods, and the number of time periods included in the second time period is not limited.

The processor 212 may determine operation result data based on a computational algorithm in one time period and then determine predicted result data using a machine learning model for a subsequent time period. The processor 212 may omit or skip a computational algorithm process by determining predicted result data using a machine learning model in the process of sequentially processing data.

For example, the processor 212 may determine the first operation result data using a computational algorithm in the first time period. The processor 212 may transmit the obtained first operation result data to the memory 214. The machine learning model may output the second predicted result data corresponding to the second operation result data in the second time period based on the transmitted first operation result data. When the second time period includes 9 time periods, the second predicted result data may include predicted result data corresponding to each of the 9 time periods. In an example, the processor 212 may obtain the first operation result data in the first time period and the predicted result data corresponding to each of the 9 time periods included in the second time period through one operation of the computational algorithm so the processor 212 may obtain result data in a total of 10 time periods. When the second time period includes 9 time periods, the processor 212 may process data about 10 times faster than when the processor 212 determines operation result data using a computational algorithm for each time period.

In the process of sequentially processing data, the processor 212 may train the machine learning model based on operation result data determined based on a computational algorithm in one time period. The processor 212 may determine whether a condition is satisfied based on the operation result data determined in the one time period. The processor 212 may determine whether a condition is satisfied for each time period, and according to examples, periodically determine whether a condition is satisfied for each of a plurality of time periods.

An operation in which the processor 212 determines operation result data based on a computational algorithm in one time period may be defined as a sequential mode. When a condition is satisfied while the processor 212 operates in the sequential mode, the processor 212 may determine predicted result data in a subsequent time period based on the operation result data determined in the previous time period and the machine learning model. An operation in which the processor 212 determines predicted result data based on a machine learning model may be defined as an acceleration mode.

The processor 212 may determine the first operation result data based on the computational algorithm in the first time period. The processor 212 may train the machine learning model based on the first operation result data. The processor 212 may determine whether a condition is satisfied based on the first operation result data. The processor 212 may simultaneously and parallelly perform an operation of training the learning model based on the first operation result data and an operation of determining whether a condition is satisfied based on the first operation result data, and the order of the two operations may change according to examples.

When a first condition is satisfied, the processor 212 may determine the second predicted result data based on the first operation result data and the machine learning model in the second time period. The second time period may include a plurality of time periods, and the second predicted result data may include predicted result data in each of the plurality of time periods.

When the first condition is not satisfied, the processor 212 may determine the second operation result data based on the first operation result data and the computational algorithm in the second time period. The processor 212 may train the machine learning model based on the second operation result data. The processor 212 may determine whether a condition is satisfied based on the second operation result data. When the operation result data determined in one time period does not satisfy the condition, the processor 212 may continue to perform in the sequential mode to determine result data for each time period based on the computational algorithm.

The processor 212 may determine a first loss value of the machine learning model based on the first operation result data. The processor 212 may compare the first loss value of the machine learning model with a threshold value. The condition may be satisfied when the first loss value is less than the threshold value. When it is determined that the first loss value is less than the threshold value, the processor 212 may operate in the acceleration mode and determine the second predicted result data based on the first operation result data and the machine learning model in the second time period.

The processor 212 may determine a second loss value of the machine learning model based on the second predicted result data. The processor 212 may determine whether a second condition is satisfied based on the second predicted result data, and when the second condition is not satisfied, the processor 212 may determine third operation result data based on the second predicted result data and the computational algorithm in a third time period.

When the second condition is satisfied, the processor 212 may determine third predicted result data corresponding to the third operation result data based on the second predicted result data and the machine learning model in the third time period. The second condition may be satisfied when the second loss value is less than the threshold value. The threshold value used to determine whether the second condition is satisfied may be the same as the threshold value used to determine whether the first condition is satisfied described above.

The processor 212 may determine the first operation result data based on the computational algorithm in the first time period and determine second predicted input data in the second time period and third predicted input data in the third time period based on the first operation result data and the machine learning model. The predicted input data may correspond to input data to calculate operation result data or result data corresponding to predicted result data. In an example, the processor 212 may determine predicted input data in a plurality of time periods after the first time period based on the machine learning model, and the amount of predicted input data to be determined is not limited.

The processor 212 may determine the second predicted result data based on the second predicted input data and the computational algorithm in the second time period and determine the third predicted result data based on the third predicted input data and the computational algorithm in the third time period. The process of determining the second predicted result data in the second time period and the process of determining the third predicted result data in the third time period may be performed simultaneously and in parallel.

The processor 212 may simultaneously and in parallel determine predicted result data based on the predicted input data for each time period by determining predicted input data in a plurality of subsequent time periods through one operation of a computational algorithm in one time period. The operation of determining predicted result data simultaneously and in parallel may significantly reduce an execution time of an entire operation process.

The second time period may include a plurality of time periods. The second predicted result data may include predicted result data corresponding to each of the plurality of time periods included in the second time period. The third time period may include a plurality of time periods. The third predicted result data may include predicted result data corresponding to each of the plurality of time periods included in the third time period.

The overall data processing speed may increase in proportion to the number of time periods included in the second time period and the third time period. This is because the amount of data processed simultaneously and in parallel may increase as the number of time periods included in the second time section and the third time section increases.

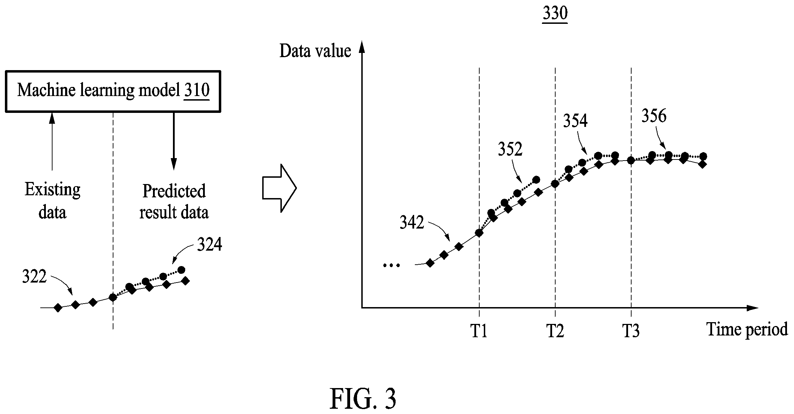

FIG. 3 illustrates an example operation of an example machine learning model, in accordance with one or more embodiments.

Referring to FIG. 3, operation result data processed by a processor (e.g., the processor 212) of a data processing apparatus (e.g., the data processing apparatus 100) may have a predetermined data pattern on a time axis as time series data. In an example, a value of the operation result data may have a tendency to decrease as a degree of a delta value (or a differential value) increases. In other words, the operation result data may be fitted or approximated to a polynomial function and current, or future data may be predicted using existing data.

Referring to FIG. 3, a machine learning model 310, executed under the control of the processor, may learn the above-described operation result data and output predicted result data from the existing data through a prediction algorithm. The machine learning model 310 may output the predicted result data from the existing data based on, for example, a polynomial approximation algorithm. However, the examples is not limited thereto.

When operation result data 322 determined by an actual computational algorithm exists, the machine learning model 310 may output predicted result data 324 corresponding to the operation result data of a predetermined time period based on existing operation result data.

A graph 330 shows an example of a change in data values of each of operation result data 342 and predicted result data 352, 354, and 356 according to the time period. The operation result data 342 (represented by a solid line) may be operation result data determined by the processor based on the computational algorithm. The predicted result data 352, 354, and 356 (represented by dotted lines) may be predicted result data that is determined by the processor based on the machine learning model 310.

Referring to the graph 330, it can be seen that the processor may determine the operation result data using the computational algorithm in time periods T1, T2, and T3, and after the operation result data is determined, the processor may generate predicted result data corresponding to the operation result data by using the machine learning model 310 for 4 time periods. In an example, the time periods T1, T2, and T3 may have a constant period. Referring to the graph 330, the processor may process data in 5 time periods through one computational algorithm process. According to the examples, 4 or more time periods may exist between the time periods T1, T2, and T3.

The processor may omit an operation to obtain operation result data corresponding to each of the 4 time periods existing between the time period T1 and the time period T2 by determining the operation result data in the time period T1 and the predicted result data in the 4 subsequent time periods using the computational algorithm in the time period T1. The processor may omit an operation to obtain operation result data corresponding to each of the 4 time periods existing between the time period T2 and the time period T3 by determining the operation result data in the time period T2 and the predicted result data in the 4 subsequent time periods using the computational algorithm in the time period T2. When the processor determines the operation result data and predicted result data using the computational algorithm every 5 time periods, compared to when the operation result data is determined based on the computational algorithm in each of all the time periods, the overall data processing speed may be substantially increased by 5 times.

The machine learning model 310 may generate unreceived data values as predicted result data based on previously received operation result data and/or previously generated predicted result data.

As described above, the processor may significantly reduce the execution time of the entire operation process by intermittently or periodically determining operation result data based on the computational algorithm instead of determining operation result data based on the computational algorithm for every time period.

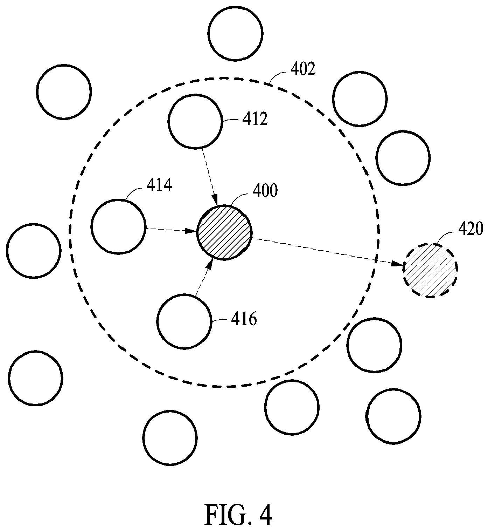

FIG. 4 illustrates an example molecular dynamics simulation using a computational algorithm, in accordance with one or more embodiments.

A computational algorithm executed under the control of a processor (e.g., the processor 212 of FIG. 2) of a data processing apparatus (e.g., the data processing apparatus 100 of FIG. 1) may be used to calculate motions of particles for molecular dynamics simulation. Molecular dynamics simulation may involve simulating a process in which multiple particles interact with each other and change over time in a 2D or 3D physical system using Newtonian dynamics. Molecular dynamics simulation may simulate a temporal change process in the entire physical system by calculating a force acting on each particle (or molecule) over time and continuously calculating a position and movement velocity of the particles.

Referring to FIG. 4, many molecules may be distributed around a molecule 400. Neighboring molecules 412, 414, and 416 within a reference range 402 among molecules around the molecule 400 may affect the motion of the molecule 400. The molecule 400 may receive a force from the neighboring molecules 412, 414, and 416, and the force received by the molecule 400 from the neighboring molecules 412, 414, and 416 may change according to a position and/or velocity of the molecule 400, and/or a velocity of the neighboring molecules 412, 414, and 416.

The processor may calculate a position and a velocity of a molecule based on a computational algorithm, and determine a force acting on the molecule corresponding to operation result data based on the position and the velocity of the molecule. The processor may calculate a position and velocity of a molecule 420 in a subsequent time period based on the operation result data.

Alternatively, the processor may determine predicted result data corresponding to the force acting on the molecule based on a machine learning model. When the processor determines the predicted result data based on the machine learning model, the accuracy of the position and velocity values of the molecule 420 in the subsequent time period may be lower than a value calculated by the processor based on the computational algorithm. When the processor determines the predicted result data based on the machine learning model, reduction of the execution time of an entire operation process and accuracy of a data result may have a trade-off relationship.

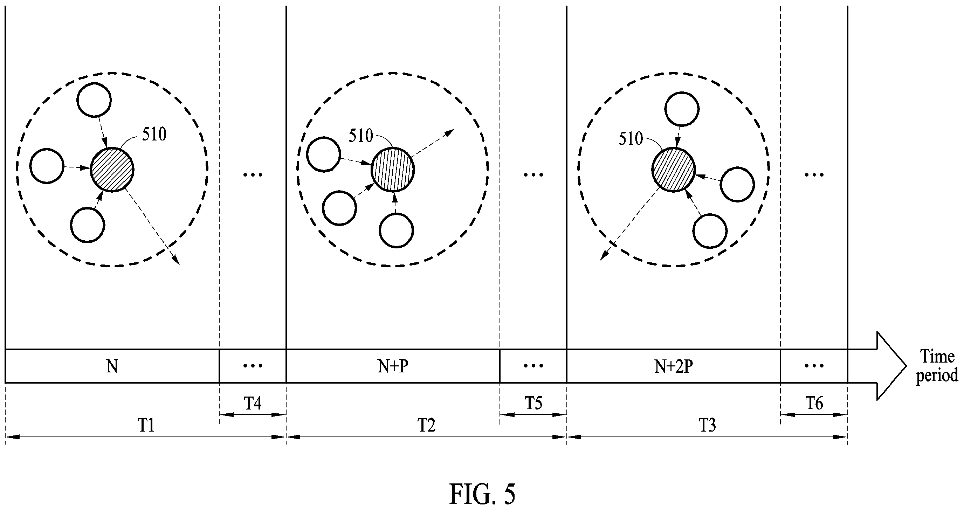

FIG. 5 illustrates an example molecular dynamics simulation, in accordance with one or more embodiments.

Molecular dynamics simulation may be performed under the control of a processor (e.g., the processor 212 of FIG. 2) of a data processing apparatus (e.g., the data processing apparatus 100 of FIG. 1). Referring to FIG. 5, an example of molecular dynamics simulation according to time periods is illustrated. Many molecules may be distributed around a molecule 510 in an Nth time period. Neighboring molecules within a reference range among molecules around the molecule 510 may affect the motion of the molecule 510.

The processor may calculate a position and a velocity of the molecule 510 in the Nth time period based on a computational algorithm, and determine a force acting on the molecule 510 corresponding to operation result data. The processor may calculate the position and velocity of the molecule 510 in the time periods from the Nth time period to before an N+Pth time period based on the operation result data. The processor may determine predicted result data corresponding to a force acting on the molecule 510 in the time periods from the Nth time period to before the N+Pth time period based on a machine learning model.

The processor may calculate a position and velocity of the molecule 510 in the N+Pth time period based on a computational algorithm and determine a force acting on the molecule 510 corresponding to operation result data. The processor may calculate the position and velocity of the molecule 510 in the time periods from the N+Pth time period to before an N+2Pth time period based on the operation result data. The processor may determine predicted result data corresponding to a force acting on the molecule 510 in the time periods from the N+Pth time period to before the N+2Pth time period based on a machine learning model.

The processor may repeatedly calculate the position and velocity of the molecule 510 using a computational algorithm in a predetermined time period, and determine the force acting on the molecule 510 corresponding to the operation result data.

By calculating the position and velocity of the molecule 510 based on a computational algorithm in the Nth time period, and determining the force acting on the molecule 510 corresponding to the operation result data, the processor may determine the force acting on the molecule 510 without using a computational algorithm in the time periods included in period T4. In other words, by determining the force acting on the molecule 510 in the time periods included in period T1 by using a single computational algorithm, the overall data processing time rate may be reduced. Period T4 may include a plurality of time periods, and the predicted result data corresponding to the force acting on the molecule 510 in the time periods from the Nth time period to before the N+Pth time period may include predicted result data corresponding to each of the plurality of time periods included in period T4.

Similar to the process in which the processor determines the force acting on the molecule 510 in the time periods included in period T1 based on a single computational algorithm in the Nth time period, the processor may determine a force acting on the molecule 510 in the time periods included in period T2 based on a single computational algorithm in the N+Pth time period and determine a force acting on the molecule 510 in the time periods included in period T3 based on a single computational algorithm in the N+2Pth time period.

The overall data processing speed may increase in proportion to the number of time periods included in periods T4 to T6. In the time periods included in periods T4 to T6, since predicted result data obtained based on the machine learning model may be used instead of the computational algorithm, the overall data processing speed may be reduced.

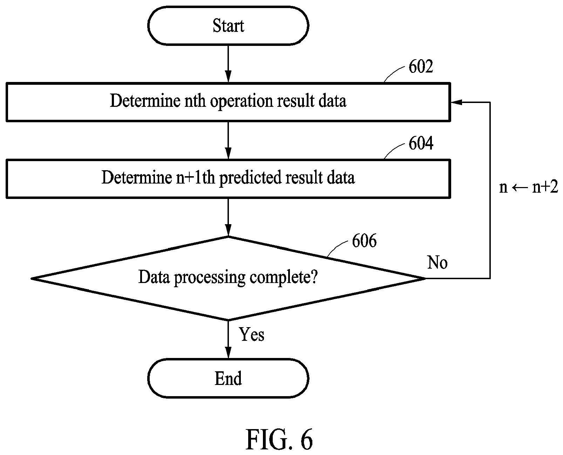

FIG. 6 illustrates an example data processing method, in accordance with one or more embodiments.

In an example, operations of the data processing method may be performed by a data processing apparatus (e.g., the data processing apparatus 100).

In operation 602, the data processing apparatus may determine nth operation result data based on a computational algorithm in an nth time period (where n is a natural number). Hereinafter, for ease of description, n may be 1. The data processing apparatus may determine first operation result data based on a computational algorithm in a first time period. The first operation result data may correspond to time series data. The data processing apparatus may calculate motions of particles for molecular dynamics simulation using the computational algorithm. An operation in which the data processing apparatus calculates the motions of particles for molecular dynamics simulation may be understood by referring to the above-described examples.

In operation 604, the data processing apparatus may determine second predicted result data based on the first operation result data and a machine learning model in a second time period. As described above with reference to FIG. 1, the second time period may include a plurality of time periods subsequent to the first time period. The second time period may include a plurality of time periods. The machine learning model may be implemented as a multi-layer perceptron, but is not limited thereto. The machine learning model may determine predicted result data based on a polynomial approximation algorithm. The predicted result data may include predicted result data corresponding to each of the plurality of time periods included in the second time period. However, an algorithm used by the machine learning model to determine the predicted result data is not limited thereto and may vary according to examples.

In operation 606, the data processing apparatus may determine whether data processing has been completed. When it is determined that the data processing has not been completed, the data processing apparatus may return to operation 602 to determine the n+2th operation result data in an n+2th time period that is a time period following an n+1th time period. In operation 606, the data processing apparatus may end the data processing operation when it is determined that the data processing has been completed. The overall data processing speed may increase in proportion to the number of time periods included in the second time period. This is because as the number of time periods included in the second time period increases, the number of times the computational algorithm is used decreases, and the data processing speed may increase as the amount of calculation decreases.

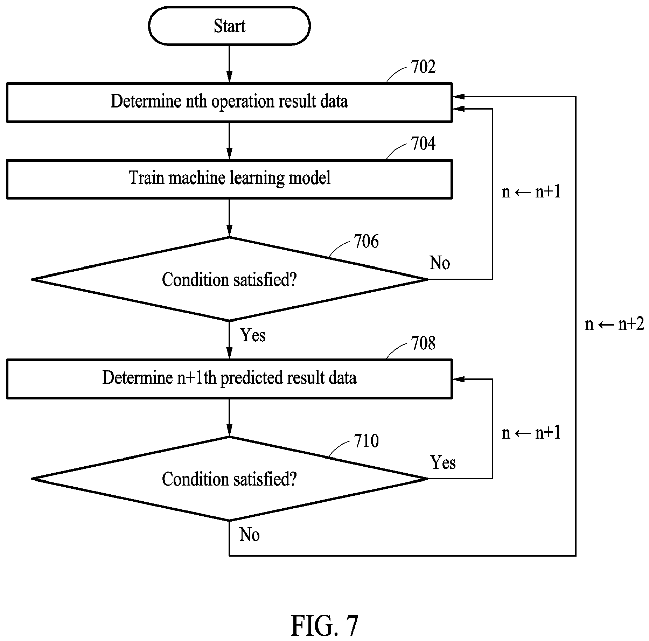

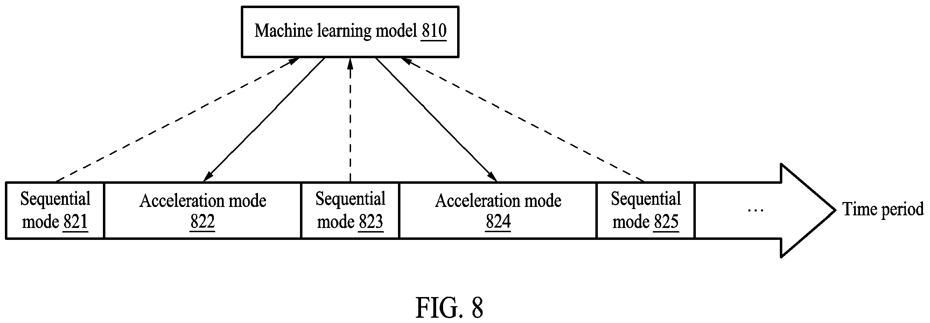

FIG. 7 illustrates an example operation of an example data processing apparatus conditionally determining predicted result data, in accordance with one or more embodiments.. FIG. 8 illustrates an example operation in which a data processing apparatus performs a sequential mode and an acceleration mode, in accordance with one or more embodiments. Hereinafter, FIGS. 7 and 8 are described together.

Referring to FIG. 7, a data processing method performed by a data processing apparatus (e.g., the data processing apparatus 100) is illustrated. Hereinafter, for ease of description, n may be 1 (where n is a natural number). However, a value of n is not limited within the range of natural numbers.

In operation 702, the data processing apparatus (e.g., the data processing apparatus 100) may determine first operation result data based on a computational algorithm in a first time period. The operation in which the data processing apparatus determines operation result data based on a computational algorithm in operation 702 may correspond to a sequential mode 821 of FIG. 8.

In operation 704, the data processing apparatus may train a machine learning model based on the first operation result data. Referring to FIG. 8, the data processing apparatus may provide the operation result data determined in the sequential mode 821 to the machine learning model to train the machine learning model.

In operation 706, the data processing apparatus may determine whether a condition is satisfied based on the first operation result data. When the condition is satisfied in operation 706, the data processing apparatus may determine second predicted result data based on the first operation result data and the machine learning model in operation 708. The operation in which the data processing apparatus determines the second predicted result data based on the first operation result data and the machine learning model may be understood by referring to the example described above with reference to FIG. 2. The operation in which the data processing apparatus determines predicted result data based on the machine learning model may correspond to an acceleration mode. The operation in which the data processing apparatus determines predicted result data when the condition is satisfied in operation 706 may correspond to an acceleration mode 822 of FIG. 8.

When the condition is not satisfied in operation 706, the data processing apparatus may return to operation 702 and determine second operation result data based on the first operation result data and the computational algorithm in the second time period. The operation of determining the second operation result data when the condition is not satisfied in operation 706 may correspond to a sequential mode 823 of FIG. 8.

The data processing apparatus may determine a first loss value of the machine learning model based on the first operation result data. The condition may be satisfied when the first loss value is less than the threshold value. The operation in which the data processing apparatus determines whether the condition is satisfied may be understood by referring to the example described above with reference to FIG. 2.

The data processing apparatus may determine a second loss value of the machine learning model based on the second predicted result data. In operation 710, the data processing apparatus may determine whether a second condition is satisfied based on the second predicted result data. When the second condition is not satisfied, the data processing apparatus may return to operation 702 and determine third operation result data based on the second predicted result data and the computational algorithm in a third time period. The operation in which the data processing apparatus returns to operation 702 to determine the third operation result data may correspond to a sequential mode 825 of FIG. 8.

When the second condition is satisfied in operation 710, the data processing apparatus may return to operation 708 and determine third predicted result data corresponding to the third operation result data based on the second predicted result data and the machine learning model in the third time period. The operation in which the data processing apparatus returns to operation 708 to determine the third predicted result data may correspond to an acceleration mode 824 of FIG. 8.

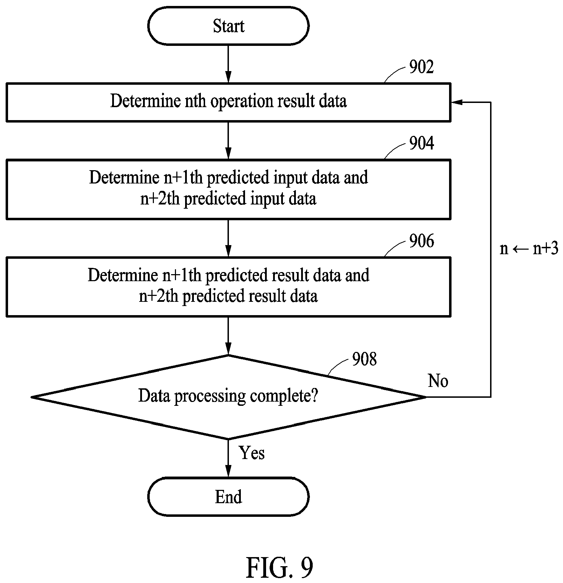

FIG. 9 illustrates an example operation of an example data processing apparatus determining multiple predicted result data in parallel, in accordance with one or more embodiments.

For example, operations of the data processing method may be performed by a data processing apparatus (e.g., the data processing apparatus 100). Hereinafter, for ease of description, n may be 1 (where n is a natural number).

In operation 902, the data processing apparatus may determine first operation result data based on a computational algorithm in a first time period.

In operation 904, the data processing apparatus may determine second predicted input data and third predicted input data based on the first operation result data and a machine learning model. The operation in which the data processing apparatus determines the second and third predicted input data in operation 904 may be understood by referring to the example described above with reference to FIG. 2.

In operation 906, the data processing apparatus may determine second predicted result data and third predicted result data based on the second predicted input data and the third predicted input data. In operation 906, an execution time of the overall operation process may be significantly reduced by the data processing apparatus determining result data simultaneously and in parallel in a plurality of time periods.

The data processing apparatus 100, first processor 112, second processor, 114, third processor, 116, fourth processor 118, computing device 200, memory 214, processor 212, communications interface device 216, and other components and devices of FIGS. 1-9, and other circuitries, components or devices described herein are implemented as, and by, hardware components. Examples of hardware components that may be used to perform the operations described in this application where appropriate include controllers, sensors, generators, drivers, memories, comparators, arithmetic logic units, adders, subtractors, multipliers, dividers, integrators, and any other electronic components configured to perform the operations described in this application. In other examples, one or more of the hardware components that perform the operations described in this application are implemented by computing hardware, for example, by one or more processors or computers. A processor or computer may be implemented by one or more processing elements, such as an array of logic gates, a controller and an arithmetic logic unit, a digital signal processor, a microcomputer, a programmable logic controller, a field-programmable gate array, a programmable logic array, a microprocessor, or any other device or combination of devices that is configured to respond to and execute instructions in a defined manner to achieve a desired result. In one example, a processor or computer includes, or is connected to, one or more memories storing instructions or software that are executed by the processor or computer. Hardware components implemented by a processor or computer may execute instructions or software, such as an operating system (OS) and one or more software applications that run on the OS, to control the operations described in this application. The hardware components may also access, manipulate, process, create, and store data in response to execution of the instructions or software. For simplicity, the singular term "processor" or "computer" may be used in the description of the examples described in this application, but in other examples multiple processors or computers may be used, or a processor or computer may include multiple processing elements, or multiple types of processing elements, or both. For example, a single hardware component or two or more hardware components may be implemented by a single processor, or two or more processors, or a processor and a controller. One or more hardware components may be implemented by one or more processors, or a processor and a controller, and one or more other hardware components may be implemented by one or more other processors, or another processor and another controller. One or more processors, or a processor and a controller, may implement a single hardware component, or two or more hardware components. A hardware component may have any one or more of different processing configurations, examples of which include a single processor, independent processors, parallel processors, single-instruction single-data (SISD) multiprocessing, single-instruction multiple-data (SIMD) multiprocessing, multiple-instruction single-data (MISD) multiprocessing, and multiple-instruction multiple-data (MIMD) multiprocessing.

The methods that perform the operations described in this application, and illustrated in FIGS. 1-9, are performed by computing hardware, for example, by one or more processors or computers, implemented as described above executing instructions or software to control the operations described in this application that are performed by the methods. For example, a single operation or two or more operations may be performed by a single processor, or two or more processors, or a processor and a controller. One or more operations may be performed by one or more processors, or a processor and a controller, and one or more other operations may be performed by one or more other processors, or another processor and another controller, e.g., as respective operations of processor implemented methods. One or more processors, or a processor and a controller, may perform a single operation, or two or more operations.

Instructions or software to control computing hardware, for example, one or more processors or computers, to control the neural network circuits or circuitry to perform the methods as described above may be written as computer programs, code segments, instructions or any combination thereof, for individually or collectively instructing or configuring the one or more processors or computers to operate as a machine or special-purpose computer to perform such control of the operations that be performed by the neural network circuits or circuitry and the methods as described above. In one example, the instructions or software include machine code that is directly executed by the one or more processors or computers, such as machine code produced by a compiler. In another example, the instructions or software include higher-level code that is executed by the one or more processors or computers using an interpreter. The instructions or software may be written using any programming language based on the block diagrams and the flow charts illustrated in the drawings and the corresponding descriptions in the specification, which disclose algorithms for performing the operations that are performed by the hardware components and the methods as described above.

The instructions or software to control computing hardware, for example, one or more processors or computers, to implement the control of hardware components to perform the methods as described above, and any associated data, data files, and data structures, may be recorded, stored, or fixed in or on one or more non-transitory computer-readable storage media, such as in memory 20 of FIG. 1. Examples of a non-transitory computer-readable storage medium include read-only memory (ROM), random-access programmable read only memory (PROM), EEPROM, RAM, DRAM, SRAM, flash memory, non-volatile memory, CD-ROMs, CD-Rs, CD+Rs, CD-RWs, CD+RWs, DVD-ROMs, DVD-Rs, DVD+Rs, DVD-RWs, DVD+RWs, DVD-RAMs, BD-ROMs, BD-Rs, BD-R LTHs, BD-REs, blue-ray or optical disk storage, hard disk drive (HDD), solid state drive (SSD), flash memory, a card type memory such as multimedia card micro or a card (for example, secure digital (SD) or extreme digital (XD)), magnetic tapes, floppy disks, magneto-optical data storage devices, optical data storage devices, hard disks, solid-state disks, and any other device that is configured to store the instructions or software and any associated data, data files, and data structures in a non-transitory manner and provide the instructions or software and any associated data, data files, and data structures to one or more processors and computers so that the one or more processors and computers can execute the instructions. In one example, the instructions or software and any associated data, data files, and data structures are distributed over network-coupled computer systems so that the instructions and software and any associated data, data files, and data structures are stored, accessed, and executed in a distributed fashion by the one or more processors or computers.

While this disclosure includes specific examples, it will be apparent to one of ordinary skill in the art, after an understanding of the disclosure of this application, that various changes in form and details may be made in these examples without departing from the scope of the claims. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner, and/or replaced or supplemented by other components or their equivalents.

Therefore, in addition to the above disclosure, the scope of the invention is defined by the enclosed claims.

## Claims

1. A data processing apparatus, comprising:
one or more processors configured to execute instructions;
a memory configured to store the instructions that, when executed by the one or more processors, configure the one or more processors to:
determine first operation result data using a computational algorithm in a first predetermined time period;
predict second predicted result data of a set second time period using a machine learning model provided the first operation result data; and
determine third operation result data using the computational algorithm in a set third time period after the second time period.

2. The apparatus of claim 1, wherein the second time period comprises a plurality of time periods,
wherein the second predicted result data comprises predicted result data corresponding to each of the plurality of time periods, and
wherein an overall data processing speed increases in proportion to a number of time periods comprised in the second time period.

3. The apparatus of claim 1 or 2, wherein the first operation result data corresponds to time series data.

4. The apparatus of one of claims 1 to 3, wherein the machine learning model generates the predicted result data based on a polynomial approximation algorithm.

5. The apparatus of one of claims 1 to 4, wherein the one or more processors are configured to:
calculate motions of particles in a molecular dynamics simulation operation based on the computational algorithm.

6. The apparatus of one of claims 1 to 5, wherein the one or more processors are configured to:
train the machine learning model based on the first operation result data; and
determine whether a first condition is satisfied based on the first operation result data,
and
the determining of the second predicted result data is performed in response to the first condition being determined to be satisfied.

7. The apparatus of claim 6, wherein the one or more processors are configured to:
determine second operation result data based on the first operation result data and the computational algorithm in the second time period in response to the first condition being determined to not be satisfied.

8. The apparatus of claim 7, wherein the one or more processors are configured to:
determine a first loss value of the machine learning model based on the first operation result data, and
the first condition is satisfied in response to the first loss value being less than a threshold value.

9. The apparatus of claim 8, wherein the one or more processors are configured to:
determine a second loss value of the machine learning model based on the second predicted result data;
determine whether a second condition is satisfied based on the second predicted result data;
determine third operation result data based on the second predicted result data and the computational algorithm in a third time period in response to the second condition being determined to not be satisfied; and
determine third predicted result data corresponding to the third operation result data based on the second predicted result data and the machine learning model in the third time period in response to the second condition being determined to be satisfied, and
the second condition is satisfied in response to the second loss value being less than the threshold value.

10. A processor-implemented data processing method performed by a data processing apparatus, the method comprising:
determining first operation result data based on a computational algorithm in a first predetermined time period;
predicting second predicted result data of a set second time period using a machine learning model provided the first operation result data; and
determining second operation result data based on the first operation data using the computational algorithm in the second time period.

11. The method of claim 10 further comprising:
predicting second predicted input data of the set second time period and third predicted input data of a set third time period, using the machine learning model provided the first operation result data;
predicting second predicted result data based on the second predicted input data and the computational algorithm in the second time period; and
predicting third predicted result data based on the third predicted input data and the computational algorithm in the third time period,
wherein the determining of the second predicted result data in the second time period, and the determining of the third predicted result data in the third time period are performed simultaneously and in parallel.

12. The method of claim 11, wherein the second time period comprises a plurality of time periods,
the second predicted result data comprises predicted result data corresponding to each of the plurality of time periods,
the third time period comprises a plurality of time periods,
the third predicted result data comprises predicted result data corresponding to each of the plurality of time periods of the third time period, and
an overall data processing speed increases in proportion to the number of time periods comprised in the second time period and the number of time periods comprised in the third time period.

13. A computer-readable storage medium storing instructions that, when executed by the one or more processors of the data processing apparatus of one of claims 1 to 9, causes the one or more processors to perform the method comprising:
determining first operation result data based on a computational algorithm in a first time period;
predicting second predicted result data of a second time period using a machine learning model provided the first operation result data, and
determining third operation result data using the computational algorithm in a set third time period after the second time period.
